# EUROPEAN PATENT SPECIFICATION

(11) **EP 3 610 771 B1**
(45) Date of publication and mention of the grant of the patent: **15.09.2021**
(21) Application number: 19177101.3
(22) Date of filing: 28.05.2019
(51) Int. Cl.: A61B 1/00, A61B 90/00, A61B 90/90, A61B 90/94

(54) **CONTROL DEVICE FOR AN ENDOSCOPE WITH MEANS FOR DETECTING A PATTERN AND FOR IDENTIFYING WHETHER THE ENDOSCOPE IS IN A NON-USE STATE OR IN A USE STATE AND AN ASSOCIATED METHOD AND A PROGRAM**
STEUERUNGSGERÄT FÜR EIN ENDOSKOP MIT MITTELN ZUR ERKENNUNG EINES MUSTERS UND ZUR FESTSTELLUNG, OB DAS ENDOSKOP IN EINEM NICHT-IN-BETRIEBSZUSTAND ODER IN EINEM IN-BETRIEBSZUSTAND IST UND EIN ZUGEHÖRIGES VERFAHREN UND EIN PROGRAMM
DISPOSITIF DE CONTRÔLE POUR UN ENDOSCOPE AVEC DES MOYENS POUR DÉTECTER UN MOTIF ET POUR IDENTIFIER SI L'ENDOSCOPE EST DANS UN ÉTAT DE NON-UTILISATION OU DANS UN ÉTAT D'UTILISATION ET UN PROCÉDÉ ASSOCIÉ ET UN PROGRAMME

(30) Priority: 16.08.2018 JP 2018153132
(43) Date of publication of application: 19.02.2020
(73) Proprietor: FUJIFILM Corporation, Tokyo 106-8620 (JP)
(72) Inventor: KAGAYA, Makoto, Ashigarakami-gun Kanagawa 258-8538 (JP)
(74) Representative: Klunker IP Patentanwälte PartG mbB

(56) References cited:
- US-A1- 2004 138 556
- US-A1- 2012 182 409
- US-A1- 2013 296 651
- US-A1- 2018 140 174

## Description

### BACKGROUND OF THE INVENTION

### 1. Field of the Invention

The present invention relates to a control device for an endoscope, an endoscope apparatus, a method of identifying the state of an endoscope, and a program for identifying the state of an endoscope.

### 2. Description of the Related Art

For various purposes, such as suppression of heat generation or improvement of the apparatus life, an endoscope apparatus having a function of identifying whether or not an endoscope is in a use state or in a non-use state (for example, refer to JP1989-303122A (JP-H01-303122A) and JP2011-072781A) or an endoscope apparatus having a function of identifying an insertion state in which an endoscope has been inserted into the body cavity or a non-insertion state in which an endoscope has not been inserted into the body cavity (for example, refer to JP2000-189383A) is known.

JP1989-303122A (JP-H01-303122A) describes an endoscope apparatus that automatically turns off a light source in a case where it is recognized that the endoscope is in a non-use state. The endoscope apparatus identifies a use state or a non-use state based on the change situation of a captured image obtained by continuously performing imaging with imaging elements of the endoscope. Specifically, the endoscope apparatus recognizes that the endoscope is in a non-use state in a case where a change in a captured image is small.

JP2011-072781A describes an endoscope apparatus that suppresses heat generation by reducing the output of at least one component, which is heated during the use of the apparatus, in a case where it is recognized that the apparatus is in a non-use state. In the endoscope apparatus, the movement of the apparatus is detected by a position sensor or an acceleration sensor provided therein, and a use state or a non-use state is identified according to the presence or absence of the movement.

JP2000-189383A describes an endoscope apparatus that reduces the amount of illumination light emitted from a light guide of an endoscope in a case where it is recognized that the endoscope is in a non-insertion state. In the endoscope apparatus, a non-insertion state or an insertion state is identified according to the brightness distribution of a captured image obtained by performing imaging with imaging elements of the endoscope. Specifically, the endoscope apparatus recognizes that the endoscope is in a non-insertion state in a case where the rate of change in the brightness distribution of the captured image is high.

US2013296651A1 discloses an endoscope system having an illumination unit which includes a modulation section configured to control a visible light source so that visible light from the visible light source is subjected to intensity modulation in a predetermined pattern, and emits modulated visible light as illumination light, a detection section configured to detect the modulated visible light, and a determination section configured to determine whether an insertion portion of an endoscope is present in an object based on a detection result of the detection section. The illumination unit is arranged outside the object, and the detection section is arranged at the insertion portion.

### SUMMARY OF THE INVENTION

As described in JP1989-303122A (JP-H01-303122A), in a method of recognizing that the endoscope is in a non-use state in a case where the change in the captured image is small, for example, in a case where the endoscope is placed at a predetermined place in the non-use state and the brightness of the place changes or a person passes through the vicinity of the place and the person is reflected in the captured image, the endoscope may be erroneously recognized as being in a use state.

In the method described in JP2011-072781A, it is necessary to provide a physical sensor inside the endoscope apparatus, which increases the manufacturing cost of the endoscope apparatus. In addition, for apparatuses that have already been released, it is not possible to cope with a simple mechanism, such as software version upgrade.

In the endoscope apparatus described in JP2000-189383A, even though it can be recognized that the endoscope is in a non-insertion state, a case of a use state in which the endoscope is gripped by hand and used, such as immediately before the start of the examination or immediately after the end of the examination, and a case of a non-use state in which the endoscope is not gripped and the endoscope is placed at a predetermined place and is in a standby state can not be distinguished. In addition, in the method of recognizing that the endoscope is in a non-insertion state in a case where the rate of change in the brightness distribution of the captured image is high, in a case where the endoscope is placed at a predetermined place and is in a non-use state and a change in the brightness of the place is small, the endoscope may be erroneously recognized as being in an insertion state (use state).

The invention has been made in view of the above circumstances, and it is an object of the invention to provide an endoscope apparatus, a method of identifying the state of an endoscope, and a program for identifying the state of an endoscope that can identify whether the endoscope is in a use state or in a non-use state while preventing an increase in the manufacturing cost of the endoscope.

In one aspect, there is provided an endoscope apparatus according to claim 1 of the appended claims.

In another aspect, there is provided a method of identifying a state of an endoscope, according to claim 4 of the appended claims.

In another aspect, there is provided a non-transitory computer readable recording medium according to claim 7 of the appended claims.

According to the invention, it is possible to provide an endoscope apparatus, a method of identifying the state of an endoscope, and a program for identifying the state of an endoscope to identify whether the endoscope is in a use state or in a non-use state while preventing an increase in the manufacturing cost of the endoscope.

### BRIEF DESCRIPTION OF THE DRAWINGS

Fig. 1 is a diagram showing the schematic configuration of an endoscope apparatus that is an embodiment of an endoscope apparatus of the invention.
Fig. 2 is a schematic diagram showing the schematic configuration of an endoscope in the endoscope apparatus shown in Fig. 1.
Fig. 3 is a schematic diagram showing the internal configuration of the endoscope apparatus shown in Fig. 1.
Fig. 4 is a diagram showing a functional block of a system controller of a processor device shown in Fig. 3.
Fig. 5 is a diagram showing an example of the imaging range of an imaging element in a state in which an endoscope is held by an endoscope holding unit.

### DESCRIPTION OF THE PREFERRED EMBODIMENTS

Hereinafter, an embodiment of the invention will be described with reference to the diagrams.

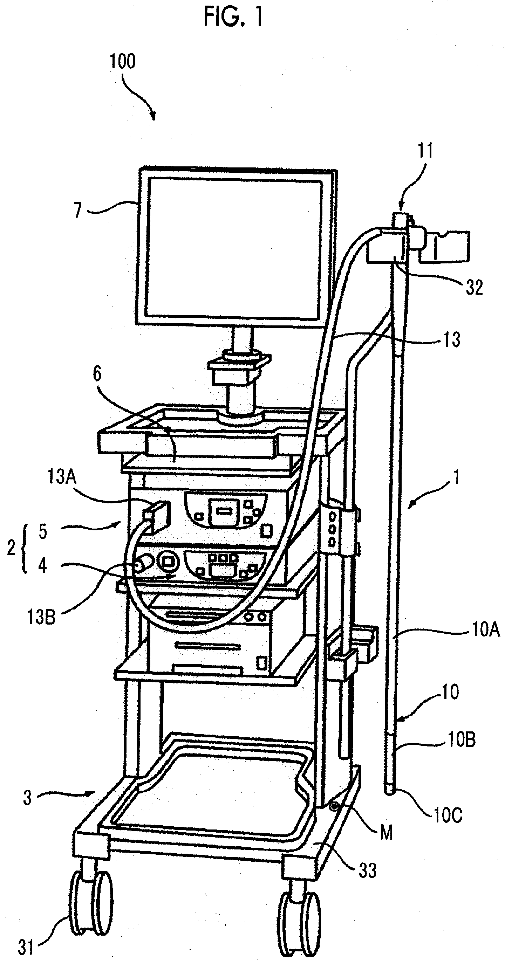

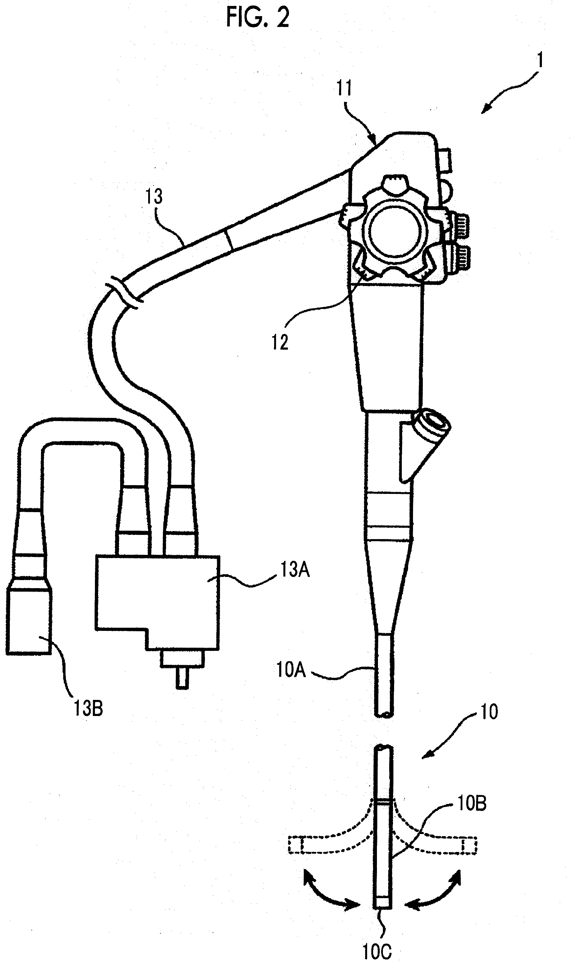

Fig. 1 is a diagram showing the schematic configuration of an endoscope apparatus 100 that is an embodiment of an endoscope apparatus of the invention. Fig. 2 is a schematic diagram showing the schematic configuration of an endoscope 1 in the endoscope apparatus 100 shown in Fig. 1.

As shown in Fig. 1, the endoscope apparatus 100 comprises an endoscope 1 and a control device 2 that controls the endoscope 1. The control device 2 comprises a processor device 4 and a light source device 5.

A display unit 7 for displaying a captured image or the like and an input unit 6, which is an interface for inputting various kinds of information to the processor device 4, are connected to the processor device 4. The processor device 4 controls the endoscope 1, the light source device 5, and the display unit 7.

As shown in Figs. 1 and 2, the endoscope 1 comprises: an insertion part 10 that is a tubular member extending in one direction, and is inserted into the body cavity; an operation unit 11 which is provided in a proximal end portion of the insertion part 10 and in which operation members for performing an observation mode switching operation, imaging and recording operations, a forceps operation, an air and water supply operation, a suction operation, an electric scalpel operation, and the like are provided; an angle knob 12 provided adjacent to the operation unit 11; and a universal cord 13 including connector portions 13A and 13B for detachably connecting the endoscope 1 to the light source device 5 and the processor device 4.

Although not shown in Fig. 2, a forceps hole for inserting biopsy forceps as a collection tool for collecting living tissue such as cells or polyps, a storage hole for storing an electric scalpel, and various channels such as air and water supply channels and a suction channel are provided inside the operation unit 11 and the insertion part 10.

The insertion part 10 is configured to include a flexible portion 10A that has flexibility, a bending portion 10B provided at the distal end of the flexible portion 10A, and a hard distal end portion 10C provided at the distal end of the bending portion 10B.

The bending portion 10B is configured so as to be able to be bent by the operation of rotating the angle knob 12. The bending portion 10B can be bent in any direction and at any angle according to a part or the like of a subject in which the endoscope 1 is used, so that the distal end portion 10C can be directed in a desired direction.

As shown in Fig. 1, the control device 2, the input unit 6, and the display unit 7 are housed in a cart unit 3 in which a caster 31 is provided on a lower base 33. The cart unit 3 forms a housing unit in which the control device 2 is housed.

An endoscope holding unit 32 for holding the endoscope 1 by suspending the operation unit 11 of the endoscope 1 is provided in the cart unit 3. With the endoscope 1 in a state of being held by the endoscope holding unit 32, the insertion part 10 always hangs down at the same position. In this state, the distal end portion 10C of the endoscope 1 faces the floor of the endoscope examination room where the cart unit 3 is provided.

The base 33 of the cart unit 3 is located closer to the floor than the distal end portion 10C of the endoscope 1 held by the endoscope holding unit 32. A marker M that is used to identify the state of the endoscope 1, which will be described later, is provided on the base 33 of the cart unit 3. The marker M is a seal on which a predetermined mark is printed, a clip on which a predetermined mark is printed, or the like. The mark has a shape that cannot be present inside the body cavity of the subject into which the insertion part 10 is inserted.

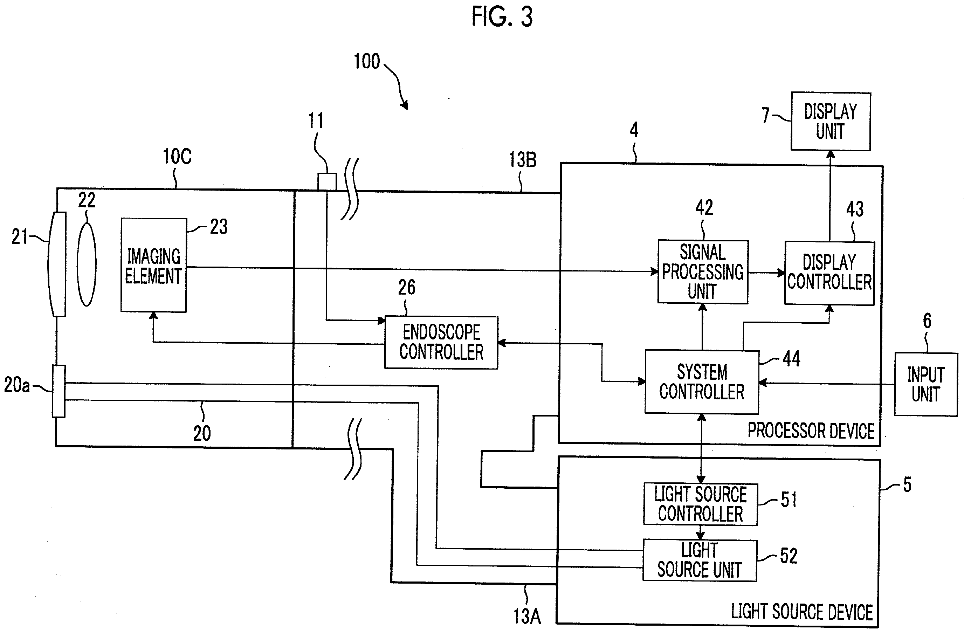

Fig. 3 is a schematic diagram showing the internal configuration of the endoscope apparatus 100 shown in Fig. 1.

The light source device 5 comprises a light source controller 51 and a light source unit 52.

The light source unit 52 generates illumination light for irradiating the subject. The illumination light emitted from the light source unit 52 is incident on a light guide 20 built in the universal cord 13, and is emitted to the subject through an illumination lens 20a provided in the distal end portion 10C of the insertion part 10.

As the light source unit 52, a white light source that emits white light or a plurality of light sources including a white light source and light sources that emit light of other colors (for example, a blue light source that emits blue light) are used. On the distal end surface of the distal end portion 10C, a plurality of illumination lenses 20a may be provided according to the type of light emitted from the light source unit 52.

The light source controller 51 includes various processors for performing processing by executing a program, and is connected to a system controller 44 of the processor device 4. The light source controller 51 controls the light source unit 52 based on a command from the system controller 44.

An imaging optical system including an objective lens 21 and a lens group 22, an imaging element 23 for imaging the subject through the imaging optical system, and the light guide 20 for guiding illumination light emitted from the light source unit 52 to the illumination lens 20a are provided in the distal end portion 10C of the endoscope 1.

The light guide 20 extends from the distal end portion 10C to the connector portion 13A of the universal cord 13. In a state in which the connector portion 13A of the universal cord 13 is connected to the light source device 5, illumination light emitted from the light source unit 52 of the light source device 5 can be incident on the light guide 20.

As the imaging element 23, it is possible to use a charge coupled device (CCD) image sensor, a complementary metal oxide semiconductor (CMOS) image sensor, and the like.

The imaging element 23 has a light receiving surface on which a plurality of pixels are arranged in a two-dimensional manner, and converts an optical image formed on the light receiving surface by the above-described imaging optical system into an electrical signal (imaging signal) in each pixel and outputs the electrical signal (imaging signal). As the imaging element 23, for example, one having a color filter of a primary color or a complementary color is used. A group of imaging signals output from the respective pixels on the light receiving surface of the imaging element 23 is referred to as a captured image signal.

In a case where the light source unit 52, which generates illumination light by splitting white light emitted from the white light source in time division using color filters of a plurality of colors, is used, the imaging element 23 in which no color filter is mounted may be used.

An endoscope controller 26 is provided inside the connector portion 13B of the universal cord 13. The endoscope controller 26 includes various processors for performing processing by executing a program. The endoscope controller 26 is connected to the system controller 44 of the processor device 4 by wiring inside the connector portion 13B. The endoscope controller 26 controls the imaging element 23 based on a command from the system controller 44. A signal corresponding to an operation (for example, an imaging operation for recording a still image) of the operation unit 11 shown in Fig. 1 is input to the endoscope controller 26.

The processor device 4 comprises a signal processing unit 42, a display controller 43, and the system controller 44.

The signal processing unit 42 generates captured image data by receiving and processing the captured image signal output and transmitted from the imaging element 23. The captured image data generated by the signal processing unit 42 is recorded on a recording medium, such as a hard disk or a flash memory (not shown).

The display controller 43 displays, on the display unit 7, a captured image based on the captured image data generated by the signal processing unit 42.

The system controller 44 controls each unit of the processor device 4, and performs overall control of the endoscope apparatus 100 by sending a command to the endoscope controller 26 of the endoscope 1 and the light source controller 51 of the light source device 5. The system controller 44 controls the imaging element 23 through the endoscope controller 26, and controls the light source unit 52 through the light source controller 51.

The system controller 44 includes various processors for performing processing by executing a program, a random access memory (RAM), and a read only memory (ROM).

The various processors in this specification include a central processing unit (CPU) that is a general-purpose processor that performs various kinds of processing by executing a program, a programmable logic device (PLD) that is a processor whose circuit configuration can be changed after manufacture, such as a field programmable gate array (FPGA), and a dedicated electric circuit that is a processor having a circuit configuration that is designed for exclusive use in order to execute specific processing, such as an application specific integrated circuit (ASIC).

More specifically, the structure of these various processors is an electric circuit in which circuit elements, such as semiconductor elements, are combined.

The system controller 44 may be configured by one of various processors, or may be a combination of two or more processors of the same type or different types (for example, a combination of a plurality of FPGAs or a combination of a CPU and an FPGA).

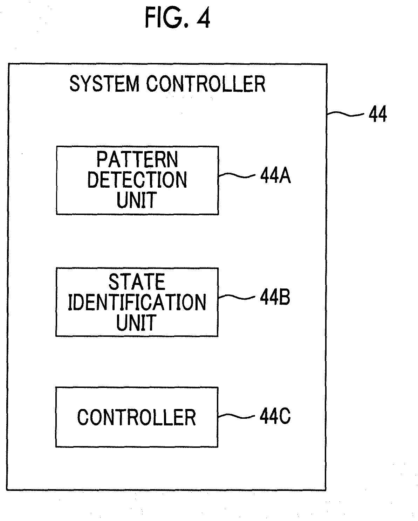

Fig. 4 is a diagram showing a functional block of the system controller 44 of the processor device 4 shown in Fig. 3.

The processor of the system controller 44 functions as a pattern detection unit 44A, a state identification unit 44B, and a controller 44C by executing a program (a program including an endoscope state identification program) stored in the ROM built in the system controller 44.

The pattern detection unit 44A acquires a captured image signal output from the imaging element 23, and detects a predetermined pattern from the acquired captured image signal.

The endoscope apparatus 100 is used on the premise that the marker M shown in Fig. 1 is disposed in the imaging range of the imaging element 23 in a state in which the endoscope 1 is held by the endoscope holding unit 32. The arrangement position of the marker M does not matter as long as the marker M is within the imaging range of the imaging element 23 in a state in which the endoscope 1 is held by the endoscope holding unit 32.

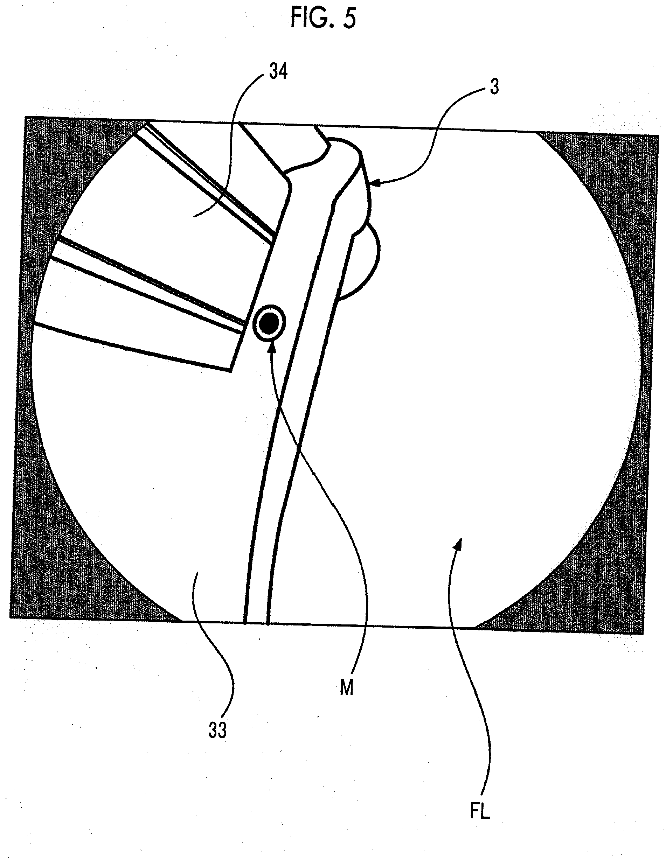

Fig. 5 is a diagram showing an example of the imaging range of the imaging element 23 in a state in which the endoscope 1 is held by the endoscope holding unit 32. As shown in Fig. 5, in the imaging range, a part of the cart unit 3 (a part of the base 33 and a part of an erected portion 34 erected on the base 33) and a part of a floor FL on which the cart unit 3 is provided are present. The cart unit 3 and the floor FL form an object in the imaging range.

Figs. 1 and 5 show an example in which the marker M is disposed on the base 33 of the cart unit 3 in the imaging range. The marker M may be disposed at a position of the floor FL in the imaging range, for example, the floor FL facing the distal end portion 10C.

The pattern detection unit 44A detects the shape of the mark printed on the marker M, as the above pattern, from the captured image signal. For example, an image of the mark printed on the marker M is stored in the ROM of the system controller 44 as a template image. Then, the pattern detection unit 44A determines the presence or absence of the above pattern by performing pattern matching using the template image from the captured image signal.

In a case where the above pattern is detected by the pattern detection unit 44A, the state identification unit 44B recognizes that the endoscope 1 is in a non-use state (a state in which the endoscope 1 is not used). In a case where the above pattern is not detected by the pattern detection unit 44A, the state identification unit 44B recognizes that the endoscope 1 is in a use state (a state in which the endoscope 1 is used).

The state in which the endoscope 1 is used refers to a state in which the endoscope 1 is detached from the endoscope holding unit 32 so that the endoscope 1 is moved by the user. The state in which the endoscope 1 is not used refers to a state in which the endoscope 1 is suspended by the endoscope holding unit 32 so that the endoscope 1 is not moved by the user.

In a case where there is a change from a state in which the state identification unit 44B recognizes that the endoscope 1 is in a non-use state to a state in which the state identification unit 44B recognizes that the endoscope 1 is in a use state, the controller 44C performs at least one of various controls exemplified in the following (A) to (G).

(A) Start emission of illumination light from the light source unit 52. (B) Start recording of a motion picture captured by the imaging element 23. (C) Start a timer that counts various times to be recorded at the time of performing an endoscope examination. (D) In a case where light in the examination room can be remotely controlled, the light is made to be dark. (E) In the case of an examination in which a colon navigation system is used, magnetic field driving of the colon navigation system is started. (F) Display information of an examinee on the display unit 7 for final checking before the examination. (G) Start a water supply or air supply pump.

The controller 44C may end the various controls shown in the above (A) to (G), for example, due to the user performing an operation on a button provided in the operation unit 11, or may end the various controls shown in the above (A) to (G) in a case where there is a change from the state in which the state identification unit 44B recognizes that the endoscope 1 is in a use state to the state in which the state identification unit 44B recognizes that the endoscope 1 is in a non-use state.

As described above, according to the endoscope apparatus 100, the above pattern can be detected from the captured image signal output from the imaging element 23, and it is possible to identify whether the endoscope 1 is in a use state or a non-use state according to the presence or absence of the pattern. Instead of identifying the use state and the non-use state using a parameter indicating the image characteristics, such as the brightness distribution of the captured image signal, by identifying the use state and the non-use state according to the detection of the pattern, the use state and the non-use state can be accurately identified regardless of the environment in which the endoscope apparatus 100 is placed.

In addition, according to the endoscope apparatus 100, even in the case of the endoscope 1 that has been shipped to the market, the use state and the non-use state of the endoscope 1 can be identified simply by providing the marker M exemplified in Figs. 1 and 5 in the cart unit 3 and updating the firmware of the system controller 44. Therefore, it is possible to construct a highly versatile system using the existing endoscope 1. Even in a case where the new endoscope 1 is sold, the endoscope 1 does not require a special mechanism. Therefore, it is possible to prevent an increase in the manufacturing cost of the endoscope 1.

In the endoscope apparatus 100, the marker M is provided in the cart unit 3 and used. Therefore, even in a case where the cart unit 3 is moved to another place, it is possible to identify the use state and the non-use state of the endoscope 1 without changing the position of the marker M.

In the above description, the marker M is provided so that one marker M is included in the imaging range shown in Fig. 5. However, the marker M may be provided so that a plurality of markers M are present in the imaging range.

For example, the marker M may be provided on each of the base 33 and the floor FL in the imaging range, or the two markers M may be provided on the base 33 in the imaging range. In this case, the pattern detection unit 44A detects each mark printed on the two markers M from the captured image signal. Then, in a case where both marks are detected, the state identification unit 44B may recognize that the endoscope 1 is in a non-use state. By providing a plurality of markers M in the imaging range described above, it can be recognized with higher accuracy that the endoscope 1 is in a non-use state.

In the above description, the pattern detection unit 44A detects the mark printed on the marker M as the above pattern. However, the pattern to be detected is not limited to the mark.

For example, the shape of the cart unit 3 (a part of the base 33 and a part of the erected portion 34) in the imaging range shown in Fig. 5 may be the above pattern. According to this configuration, since the marker M is not necessary, it is possible to reduce the manufacturing cost, the delivery cost, and the like of the marker M. In addition, according to this configuration, even in a case where the cart unit 3 is moved to another place, it is possible to identify the use state and the non-use state of the endoscope 1.

Alternatively, both the shape of the cart unit 3 in the imaging range shown in Fig. 5 and the mark printed on the marker M may be the above pattern. In this manner, it can be recognized with higher accuracy that the endoscope 1 is in a non-use state.

Although a flexible endoscope 1 is used in the endoscope apparatus 100 described so far, the invention can be similarly applied to an endoscope apparatus using a rigid endoscope.

### Explanation of References

- 100:: endoscope apparatus
- 1:: endoscope
- 2:: control device
- 20:: light guide
- 20a:: illuminating lens
- 21:: objective lens
- 22:: lens group
- 23:: imaging element
- 26:: endoscope controller
- 3:: cart unit
- 32:: endoscope holding unit
- 33:: base
- 34:: erected portion
- 4:: processor device
- 42:: signal processing unit
- 43:: display controller
- 44:: system controller
- 44A:: pattern detection unit
- 44B:: state identification unit
- 44C:: controller
- 5:: light source device
- 51:: light source controller
- 52:: light source unit
- 6:: input unit
- 7:: display unit
- 10:: insertion part
- 10A:: flexible portion
- 10B:: bending portion
- 10C:: distal end portion
- 11:: operation unit
- 12:: angle knob
- 13: universal cord
- 13A, 13B:: connector portion
- M:: marker
- FL:: floor

## Claims

1. An endoscope apparatus (100), comprising:
an endoscope (1) including an imaging element (23), and
a control device (2) for the endoscope, the control device (2) being configured to be housed in a housing unit (3) and comprising
a pattern detection unit (44A) configured to acquire a captured image signal, which is output from the imaging element (23) included in the endoscope, (1) and configured to detect a predetermined pattern from the captured image signal; and
a state identification unit (44B) configured to recognize that the endoscope (1) is in a non-use state in a case where the pattern is detected and configured to recognize that the endoscope (1) is in a use state in a case where the pattern is not detected;
wherein, in a state in which the endoscope (1) is held by an endoscope holding unit (32) for holding the endoscope provided in the housing unit (3), the detected pattern is (i) a shape of a mark (M) disposed on an object present in an imaging range of the imaging element (23) and/or (ii) a shape of the housing unit present in the imaging range.

2. The control device for an endoscope according to claim 1,
wherein the detected pattern is a shape of a first mark (M) disposed on part of the housing unit.

3. The control device for an endoscope according to claim 1 or 2,
wherein the detected pattern is a shape of a second mark (M) disposed on a floor on which the housing unit is positioned.

4. A method of identifying a state of an endoscope of an endoscope apparatus (100) including a control device (2), the control device (2) being configured to be housed in a housing unit (3), the method comprising:
a pattern detection step of acquiring a captured image signal, which is output from an imaging element (23) included in the endoscope (1), and detecting a predetermined pattern from the captured image signal; and
a state identification step of recognizing that the endoscope is in a non-use state in a case where the pattern is detected and recognizing that the endoscope (1) is in a use state in a case where the pattern is not detected.
wherein, in a state in which the endoscope (1) is held by an endoscope holding unit (32) for holding the endoscope (1) provided in the housing unit, the pattern is (i) a shape of a mark (M) disposed on an object present in an imaging range of the imaging element (23) , and/or (ii) a shape of the housing unit present in the imaging range.

5. The method of identifying a state of an endoscope according to claim 4,
wherein the detected pattern is a shape of a first mark (M) disposed on part of the housing unit.

6. The method of identifying a state of an endoscope according to claim 4 or 5,
wherein the detected pattern is a shape of a second mark (M) disposed on a floor on which the housing unit is positioned.

7. A non-transitory computer readable recording medium storing a program for identifying a state of an endoscope of an endoscope apparatus (100) including a control device (2), the control device (2) being configured to be housed in a housing unit (3), the program causing a computer to execute:
a pattern detection step of acquiring a captured image signal, which is output from an imaging element (23) included in the endoscope (1), and detecting a predetermined pattern from the captured image signal; and
a state identification step of recognizing that the endoscope (1) is in a non-use state in a case where the pattern is detected and recognizing that the endoscope (1) is in a use state in a case where the pattern is not detected.
wherein, in a state in which the endoscope (1) is held by an endoscope holding unit (32) for holding the endoscope (1) provided in the housing unit, the pattern is (i) a shape of a mark (M) disposed on an object present in an imaging range of the imaging element (23) or (ii) a shape of the housing unit present in the imaging range.

## Patentansprüche

1. Endoskopvorrichtung (100), umfassend:
ein Endoskop (1), das ein Bildgebungselement (23) enthält, und
eine Steuereinrichtung (2) für das Endoskop, welche konfiguriert ist zur Aufnahme in einer Gehäuseeinheit (3) und aufweist:
eine Musterdetektoreinheit (44A), konfiguriert zum Erfassen eines aufgenommenen Bildsignals, das von dem in dem Endoskop (1) enthaltenen Bildgebungselement (23) ausgegeben wird, und konfiguriert ist zum Detektieren eines vorbestimmten Musters aus dem aufgenommenen Bildsignal; und
eine Zustandsidentifikationseinheit (44B), konfiguriert zum Erkennen, dass sich das Endoskop (1) in einem Nicht-Gebrauchszustand befindet, falls das Muster detektiert wird und konfiguriert ist zum Erkennen, dass das Endoskop (1) sich in einem Gebrauchszustand befindet, falls das Muster nicht detektiert wird;
wobei in einem Zustand, in welchem das Endoskop (1) von einer Endoskophalteeinheit (32) zum Halten des in der Gehäuseeinheit (3) vorhandenen Endoskops gehalten wird, das detektierte Muster (i) eine Form einer Markierung (M) aufweist, die sich an einem in einem Bildbereich des Bildgebungselements (23) befindlichen Objekt befindet, und/oder (ii) eine Form der in dem Bildbereich vorhandenen Gehäuseeinheit ist.

2. Steuereinrichtung für ein Endoskop nach Anspruch 1,
bei der das detektierte Muster eine Form einer ersten Markierung (M) ist, die sich auf einem Teil der Gehäuseeinheit befindet.

3. Steuereinrichtung für ein Endoskop nach Anspruch 1 oder 2,
bei der das detektierte Muster eine Form einer zweiten Markierung (M) ist, die sich auf einem Boden befindet, auf dem die Gehäuseeinheit positioniert ist.

4. Verfahren zum Identifizieren eines Zustands eines Endoskops einer Endoskopvorrichtung (100), die eine Steuereinrichtung (2) enthält, welche konfiguriert ist zur Aufnahme in einer Gehäuseeinheit (3), umfassend:
einen Musterdetektierschritt des Erfassens eines aufgenommenen Bildsignals, das von einem in dem Endoskop (1) enthaltenen Bildgebungselement (23) ausgegeben wird, und des Detektierens eines vorbestimmten Musters aus dem aufgenommenen Bildsignals; und
einen Zustandsidentifikationsschritt des Erkennens, dass sich das Endoskop in einem Nicht-Gebrauszustand befindet, falls das Muster detektiert wird, und des Erkennens, dass sich das Endoskop (1) in einem Gebrauchszustand befindet, falls das Muster nicht detektiert wird,
wobei in einem Zustand, in welchem das Endoskop (1) von einer zum Halten des in der Gehäuseeinheit vorhandenen Endoskops (1) den in der Endoskophalteeinheit (32) gehalten wird, das Muster (i) eine Form einer Markierung (M) ist, die sich an dem in einem Bildgebungsbereich des Bildgebungselements (23) befindlichen Objekt befindet, und/oder (ii) eine Form der Gehäuseeinheit ist, die sich in dem Bildbereich befindet.

5. Verfahren zum Identifizieren eines Zustands eines Endoskops nach Anspruch 4,
bei dem das detektierte Muster eine Form einer ersten Markierung (M) ist, die sich auf einem Teil der Gehäuseeinheit befindet.

6. Verfahren zum Identifizieren eines Zustands eines Endoskops nach Anspruch 4 oder 5,
bei dem das detektierte Muster eine Form einer zweiten Markierung (M) ist, die sich auf einem Boden befindet, auf dem die Gehäuseeinheit positioniert ist.

7. Nicht-flüchtiges computer-lesbares Aufzeichnungsmedium, das ein Programm zum Identifizieren eines Zustands eines Endoskops einer Endoskopvorrichtung (100) speichert, das eine Steuereinrichtung (2) enthält, die konfiguriert ist zur Aufnahme in einer Gehäuseeinheit (3), wobei das Programm einen Computer veranlasst, folgendes auszuführen:
einen Musterdetektierschritt des Erfassens eines aufgenommenen Bildsignals, das von einem in dem Endoskop (1) enthaltenen Bildgebungselement (23) ausgegeben wird, und des Detektierens eines vorbestimmten Musters aus dem aufgenommenen Bildsignals; und
einen Zustandsidentifikationsschritt des Erkennens, dass sich das Endoskop (1) in einem Nicht-Gebrauszustand befindet, falls das Muster detektiert wird, und des Erkennens, dass sich das Endoskop (1) in einem Gebrauchszustand befindet, falls das Muster nicht detektiert wird,
wobei in einem Zustand, in welchem das Endoskop (1) von einer zum Halten des in der Gehäuseeinheit vorhandenen Endoskops (1) den in der Endoskophalteeinheit (32) gehalten wird, das Muster (i) eine Form einer Markierung (M) ist, die sich an dem in einem Bildgebungsbereich des Bildgebungselements (23) befindlichen Objekt befindet, und/oder (ii) eine Form der Gehäuseeinheit ist, die sich in dem Bildbereich befindet.

## Revendications

1. Appareil d'endoscope (100), comprenant :
un endoscope (1) incluant un élément d'imagerie (23), et
un dispositif de commande (2) pour l'endoscope, le dispositif de commande (2) étant configuré pour être logé dans une unité de logement (3) et comprenant
une unité de détection de motif (44A) configurée pour acquérir un signal d'image capturée, lequel est produit à partir de l'élément d'imagerie (23) inclus dans l'endoscope (1), et configuré pour détecter un motif prédéterminé à partir du signal d'image capturée, et
une unité d'identification d'état (44B) configurée pour reconnaître que l'endoscope (1) se trouve dans un état de non-utilisation dans un cas où le motif est détecté, et configurée pour reconnaître que l'endoscope (1) se trouve dans un état d'utilisation dans un cas où le motif n'est pas détecté ;
dans lequel dans un état où l'endoscope (1) est retenu par une unité de retenue d'endoscope (32) pour retenir l'endoscope prévu dans l'unité de logement (3). le motif détecté est (i) une forme d'un repère (M) disposé sur un objet présent dans une plage d'imagerie de l'élément d'imagerie (23) et/ou (ii) une forme de l'unité de logement présente dans la plage d'imagerie.

2. Dispositif de commande pour un endoscope selon la revendication 1,
dans lequel le motif détecté est une forme d'un premier repère (M) disposé sur une partie de l'unité de logement.

3. Dispositif de commande pour un endoscope selon la revendication 1 ou 2,
dans lequel le motif détecté est une forme d'un second repère (M) disposé sur un plancher sur lequel l'unité de logement est positionnée.

4. Procédé destiné à identifier un état d'un endoscope d'un appareil d'endoscope (100) incluant un dispositif de commande (2), le dispositif de commande (2) étant configuré pour être logé dans une unité de logement (3), le procédé comprenant les étapes suivantes :
une étape de détection de motif pour acquérir un signal d'image capturée, lequel est produit à partir d'un élément d'imagerie (23) inclus dans l'endoscope (1), et détecter un motif prédéterminé à partir du signal d'image capturée, et
une étape d'identification d'état pour reconnaître que l'endoscope se trouve dans un état de non-utilisation dans un cas où le motif est détecté et reconnaître que l'endoscope (1) se trouve dans un état d'utilisation dans un cas où le motif n'est pas détecté ;
dans lequel dans un état où l'endoscope (1) est retenu par une unité de retenue d'endoscope (32) pour retenir l'endoscope (1) prévu dans l'unité de logement, le motif est (i) une forme d'un repère (M) disposé sur un objet présent dans une plage d'imagerie de l'élément d'imagerie (23) et/ou (ii) une forme de l'unité de logement présente dans la plage d'imagerie.

5. Procédé destiné à identifier un état d'un endoscope selon la revendication 4.
dans lequel le motif détecté est une forme d'un premier repère (M) disposé sur une partie de l'unité de logement.

6. Procédé destiné à identifier un état d'un endoscope selon la revendication 4 ou 5,
dans lequel le motif détecté est une forme d'un second repère (M) disposé sur un plancher sur lequel l'unité de logement est positionnée.

7. Support d'enregistrement lisible par ordinateur non transitoire stockant un programme pour identifier un état d'un endoscope d'un appareil d'endoscope (100) incluant un dispositif de commande (2), le dispositif de commande (2) étant configuré pour être logé dans une unité de logement (3), le programme faisant en sorte qu'un ordinateur exécute :
une étape de détection de motif pour acquérir un signal d'image capturée, lequel est produit à partir d'un élément d'imagerie (23) inclus dans l'endoscope (1), et détecter un motif prédéterminé à partir du signal d'image capturée, et
une étape d'identification d'état pour reconnaître que l'endoscope (1) se trouve dans un état de non-utilisation dans un cas où le motif est détecté et reconnaître que l'endoscope (1) se trouve dans un état d'utilisation dans un cas où le motif n'est pas détecté ;
dans lequel dans un état où l'endoscope (1) est retenu par une unité de retenue d'endoscope (32) pour retenir l'endoscope (1) prévu dans l'unité de logement, le motif est (i) une forme d'un repère (M) disposé sur un objet présent dans une plage d'imagerie de l'élément d'imagerie (23) et/ou (ii) une forme de l'unité de logement présente dans la plage d'imagerie.
